# EUROPEAN PATENT APPLICATION

(11) **EP 4 502 964 A1**
(43) Date of publication of application: **05.02.2025**
(21) Application number: 23189706.7
(22) Date of filing: 04.08.2023
(51) Int. Cl.: G06V 10/77, G06V 10/764, G06V 10/82, G06V 10/774

(54) **PROVIDING A SIMILAR MEDICAL IMAGE**

(71) Applicant: Siemens Healthineers AG, 91301 Forchheim (DE)
(72) Inventor: TEICHMANN, Marvin, 91054 Erlangen (DE); GHESU, Florin-Cristian, 91083 Baiersdorf (DE); BRENDTKE, Rico, 91058 Erlangen (DE); LIPPOK, Svenja, 91080 Uttenreuth (DE); HEIMANN, Tobias, 91058 Erlangen (DE)
(74) Representative: Siemens Healthineers Patent Attorneys

(57) **Abstract**

The invention relates to a method and a system for providing a similar medical image. The methods and the system comprise receiving a first medical image related to a first patient. The methods and the system comprise furthermore comprise determining a first feature vector by applying a first machine learning model to the first medical image. The first machine learning model was trained based on training datasets, each training dataset comprising a training medical image and related non-imaging data. The methods and the system comprise furthermore comprise receiving a plurality of second feature vectors. The plurality of second feature vectors is the result of applying the first machine learning model to a plurality of second medical images. The methods and the system comprise furthermore comprise determining, based on the first feature vector, a similar feature vector from the plurality of second feature vectors. The methods and the system comprise furthermore comprise selecting the similar medical image from the plurality of second medical images, wherein the similar medical image is related to the similar feature vector. The methods and the system comprise furthermore comprise providing the similar medical image.

## Description

Independent of the grammatical term usage, individuals with male, female or other gender identities are included within the term.

Finding similar patients is an important topic in medical technology, in particular, in the context of cancer diagnosis and treatment planning, particularly when faced with rare or unusual cases. For example, identifying patients with comparable molecular makeup and tumor characteristics is crucial in guiding medical professionals towards effective treatment options and enhancing clinical decision-making. In such complex cases, standard protocols may not be sufficient, and medical professionals may struggle with uncertainty regarding the best course of action. In particular, it would be desirable to be able to find similar patients based on comparing respective medical images, for example, histopathology slide images.

Historically, pathologists have mainly depended on manual examination of histopathology slides under a microscope to identify and compare patterns and features within the tumor area. The findings are typically discussed in tumor board meetings that include medical experts from various fields, where additional data and modalities are considered. The tumor board can also be used for identifying similar patients. This approach, however, is labor-intensive, time-consuming, and can be prone to inter- and intra-observer variability, which may lead to inconsistencies in patient assessment and treatment decision-making.

Content-based image retrieval systems have been adopted to find analogous images in medical databases using global features like color, texture, and shape. While these systems have proven useful in certain cases, they might not efficiently match patients based on local patterns and features within the tumor area, and they lack data integration related to other factors to guide the retrieval process. In digital pathology, these systems mainly depend on visual features and do not incorporate other data, possibly resulting in retrieved images that visually resemble each other but represent diagnostically distinct cancers.

It is the problem of the present invention to improve finding similar patients based on medical images.

The problem is solved according to the independent claims. Further advantageous embodiments and additional advantageous features are described in the dependent claims and in the specification.

In the following, the solution according to the invention is described with respect to the claimed systems as well as with respect to the claimed methods. Features, advantages or alternative embodiments herein can be assigned to the other corresponding claimed objects and vice versa. In other words, the systems can be improved with features described or claimed in the context of the corresponding method. In this case, the functional features of the methods are embodied by objective units of the systems.

Furthermore, in the following the solution according to the invention is described with respect to methods and systems for providing a similar medical image as well as with respect to methods and systems for providing a trained machine learning model. Features, advantages or alternative embodiments herein can be assigned to the other claimed objects and vice versa. In other words, claims and embodiments for providing a trained machine learning model can be improved with features described or claimed in the context of providing a similar medical image. In particular, datasets used in the methods and systems for providing a similar medical image can have the same properties and features as the corresponding datasets used in the methods and systems for providing a trained machine learning model, and the trained machine learning models provided by the respective methods and systems can be used in the methods and systems for providing a similar medical image.

In the following, the term "in particular" is used to indicate an optional and/or advantageous additional feature. Furthermore, the terms "applying a model to data" or "applying a unit to data" is used to indicate that the respective data is used as input data for the model or the unit, or that input data is used that comprises the respective data (and potentially other data).

In a first aspect the invention is related to a computer-implemented method for providing a similar medical image. The method comprises receiving a first medical image related to a first patient. The method furthermore comprises determining a first feature vector by applying a first machine learning model to the first medical image. The first machine learning model was trained based on training datasets, each training dataset comprising a training medical image and related non-imaging data. The method furthermore comprises receiving a plurality of second feature vectors. The plurality of second feature vectors is the result of applying the first machine learning model to a plurality of second medical images. The method furthermore comprises determining, based on the first feature vector, a similar feature vector from the plurality of second feature vectors. The method furthermore comprises selecting the similar medical image from the plurality of second medical images, wherein the similar medical image is related to the similar feature vector. The method furthermore comprises providing the similar medical image. In particular, providing the similar medical image can comprise storing, transmitting and/or displaying the similar medical image.

In particular, the steps of receiving the first medical image and the step of receiving the plurality of second feature vectors are executed by an input interface, in particular, by an input interface of an image providing system. In particular, the steps of determining the first feature vector, determining, based on the first feature vector, determining the similar feature vector and selecting the similar medical image are executed by a computation unit, in particular, by a computation unit of the image providing system. In particular, the step of providing the similar medical image is executed by an output interface, in particular, by an output interface of the image providing system.

In particular, a medical image is an image of a patient recorded by a medical imaging modality. A medical image can be an X-ray image, a computed tomography image (acronym "CT image"), a magnetic resonance image (acronym "MR image"), a positron emission tomography image (acronym "PET image"), a single-photon emission computed tomography (acronym "SPECT image"), and/or an ultrasound image (acronym "US image"). Furthermore, a medical image can be a microscopy image, histopathology image and/or a time-continuous biosignal analysis image. In particular, a medical image can be a whole-slide image.

In particular, a medical image can be a two-dimensional image, a three-dimensional image or a four-dimensional image. In particular, within a four-dimensional image, there are three spatial and one time dimensions.

A medical image can comprise a plurality of pixels or voxels, wherein the term "pixel" is mainly used for the building blocks of two-dimensional images, and the term is "voxel" is used for the building blocks of images with arbitrary dimension (mainly for three and more dimensions). However, in the following, the term "pixel" will be used as synonym for the term "pixel and/or voxel". Each pixel can comprise at least one intensity value corresponding to a certain tissue property (e.g., Hounsfield units for a CT image).

A medical image can be identical with or encapsulated in one or more DICOM files. Whenever DICOM is mentioned herein, it shall be understood that this refers to the "Digital Imaging and Communications in Medicine" (DICOM) standard, for example according to the current DICOM PS3.1 2020c standard (or any later or earlier version of said standard). It is also possible that several medical images are encapsulated in a single DICOM file.

In particular, a medical image can comprise additional metadata, e.g., a patient identifier, a patient name, an identifier of the modality used to acquire the medical image, a date and time of the image acquisition, an imaging protocol used during the acquisition of the medical image, and/or other metadate related to the medical image and/or its acquisition process.

In particular, the first medical image, the second medical images, the similar medical image and the training medical image are medical images of the same type, i.e., the first medical image, the second medical images, the similar medical image and the training medical image were recorded with the same type of imaging modality (e.g., all medical images were recorded by an CT, or all medical images are whole-slide images). In particular, the first medical image, the second medical images, the similar medical image and the training medical image can have the same dimensionality. In particular, the first medical image, the second medical images, the similar medical image and the training medical image can have the same size (e.g., measured in number of pixels) along each of their dimensions. In particular, the set of second medical images can comprise the set of training medical images, and/or the set of second medical images can be identical with the set of training medical images.

In general, a machine learning model mimics cognitive functions that humans associate with other human minds. In particular, by training based on training data the machine learning model is able to adapt to new circumstances and to detect and extrapolate patterns. Another term for "machine learning model" is "trained function".

In general, parameters of a machine learning model can be adapted by means of training. In particular, supervised training, semi-supervised training, unsupervised training, reinforcement learning and/or active learning can be used. Furthermore, representation learning (an alternative term is "feature learning") can be used. In particular, the parameters of the machine learning models can be adapted iteratively by several steps of training. In particular, within the training a certain cost function can be minimized. In particular, within the training of a neural network the backpropagation algorithm can be used.

In particular, a machine learning model can comprise a neural network, a support vector machine, a decision tree and/or a Bayesian network, and/or the machine learning model can be based on k-means clustering, Q-learning, genetic algorithms and/or association rules. In particular, a neural network can be a deep neural network, a convolutional neural network or a convolutional deep neural network. Furthermore, a neural network can be an adversarial network, a deep adversarial network and/or a generative adversarial network.

In the context of this disclosure, the phrase "applying a machine learning model to a medical image" is to be understood that there might be additional data that can be used as input to the machine learning model together with the medical image. Furthermore, in the context of this disclosure, the phrase "applying a machine learning model to a medical image" is to be understood that at least parts of the medical images are used as input for the machine model (potentially together with additional data). In other words, the phrase "applying a machine learning model to a medical image" is to be interpreted as "applying a machine learning model to input data, wherein the input data comprises at least parts of a medical image".

In general, a feature is an individual measurable property or characteristic of a phenomenon. In the context of this disclosure, a feature can be an individual measurable property or characteristic related to a medical image. In particular, a feature vector is an n-dimensional vector of numerical features that represent the same object (the vector space associated with these vectors can be denoted as "feature space"). In the context of this disclosure, a feature vector can be an n-dimensional vector of numerical features that represent a medical image. In the context of this disclosure, every ordered set of numerical values can be interpreted as feature vector.

In particular, the first feature vector, the similar feature vector and the second feature vectors can have the same dimensionality. In other words, the first feature vector, the similar feature vector and the second feature vectors can be element of the same feature space.

In particular, non-imaging data is medical data. In particular, non-imaging data is medical data related to a patient. In particular, non-imaging data is medical data that does not comprise a medical image. However, non-imaging data can be derived from a medical image (e.g., a diagnosis derived by a radiologist or pathologist based on a medical image). In particular, non-imaging data can comprise at least one numerical value and/or can be mapped to at least one numerical value. In particular, non-imaging data can be an m-dimensional vector, each value of the m-dimensional vector relating to a certain patient.

The inventors have recognized that by using features vectors created by a first machine learning model trained on pairs of imaging and non-imaging data for searching similar images or similar patients, more relevant similar images can be determined. This is due to the fact that the non-imaging data can be incorporated into the parameters of the first machine learning model within training, so that the features vectors extracted by the first machine learning model are configured (by the training process) to be predictive not (only) for the visual impression of medical images, but for the respective non-imaging data of the same patient.

In particular, if using the proposed method for finding similar images or similar patients related to a certain disease (e.g., a certain type of cancer), based on the proposed training the first machine learning model can proficiently identify diagnostically relevant morphological features and patterns, and accurately retrieve patients with diseases exhibiting similarities in both morphology and non-imaging makeup. This advancement offers significant clinical benefits and usefulness, as it enables healthcare professionals to determine personalized treatment options, bolster diagnostic precision, and ultimately enhance patient outcomes, particularly in rare and complex diseases.

According to a further possible aspect of the invention, each second feature vector of the plurality of second feature vectors is the result of applying the first machine learning model to exactly one second medical image of the plurality of second medical images. In particular, there can be a one-to-one correspondence between the plurality of second feature vectors and the plurality of second medical images.

According to a further possible aspect of the invention the non-imaging data has a different structure than the first feature vector. In particular, the non-imaging data has a lower dimensionality than the first feature vector. In particular, the non-imaging data has a lower dimensionality than the first feature vector if both the non-imaging data and the first feature vector can be interpreted as vectors of numerical values, and the dimensionality of the vector representing the non-imaging data is smaller than the dimensionality of the feature vector or the vector representing the feature vector.

According to a further aspect of the invention the first medical image is a histopathology slide image, particularly a whole-slide image.

Histopathology slide images can, in particular, be two-dimensional pixel images. A histopathology slide image can map a at least parts of a tissue slide that has been prepared from a tissue sample of the patient. The preparation of tissue slides from a tissue sample can comprise the cutting out of a section (also called a 'punch biopsy' or a 'block') from the tissue sample (with a punch tool for example), with the section being cut into micrometer-thick slices, the tissue slides. The preparation of the tissue slides can further comprise the staining of the tissue slides with a histopathological staining. The staining in this case can serve to highlight different structures in the tissue slide, such as e.g. cell walls or cell nuclei or to check a medical indication, such as e.g. a cell proliferation level. In this process different histopathological stainings can be used for different issues.

For creation of the one or more histopathology slide images the tissue slides stained with the first histopathological staining can be or can have been digitalized or scanned. To this end the first tissue slides are imaged with a suitable digitalization station, such as for example what is known as a whole-slide scanner, which preferably scans the entire tissue slide mounted on the object carrier and converts it into a pixel image. In order to obtain the staining effect through the histopathological staining, the pixel images are preferably color pixel images. Since in the diagnosis both the overall impression of the tissue and also the finely resolved cell structure is of significance, the histopathology slide images typically have a very high pixel resolution. The histopathology slide images can subsequently be digitally processed and in particular archived in a suitable database. Microscopically the histopathology slide images show the fine tissue structure of the tissue sample and in particular the cell structure or the cells contained in the tissue sample. On greater length scales the histopathology slide images show superordinate tissue structures and features, such as e.g. the density and cell morphologically contiguous regions, such as e.g. tumor regions or stroma regions.

In particular, the first medical image is a histopathology slide image depicting a tissue slice of the patient stained with a histopathological stain. In particular, the histopathological stain can be a H&E (acronym for "hematoxylin and eosin") stain or a IHC (acronym for "immunohistochemistry") stain.

In H&E stains, hematoxylin stains cell nuclei, and eosin stains the extracellular matrix and cytoplasm. H&E stain is the most widely used stain in digital pathology, making the method of providing similar images widely applicable.

IHC stains involve the process of selectively identifying antigens (proteins) in cells of a tissue section by exploiting the principle of anti-bodies binding specifically to antigens in biological tissues. With that, structures can be highlighted which are not accessible with other stains such as H&E, and which might be highly relevant in the context of a specific disease a certain similar patient shall be retrieved.

In particular, the IHC stain may comprise biomarkers (e.g., in the form of antibodies) configured to target (cytoskeletal) keratins. Keratins form part of the cell cytoskeleton and define the mechanical properties of cells. As such, the abundance of keratins makes up a good tumor marker as keratin ex-pression levels are often altered in tumor cells. Accordingly, using keratin IHC stains may enable to identify adenocarcinoma (neoplasia of epithelial tissue). Specifically, the IHC stain may comprise keratin biomarkers targeting different keratin forms such as CK-5, CK-8, CK-14, CK-18 (wherein `CK' stands for 'cytoskeletal keratin'). Self-speaking, the IHC stain may comprise different or additional biomarkers such as p63 and AMACR biomarkers.

A while-slide image is a histopathology image that maps a whole tissue slide that has been prepared from a tissue sample of the patient. Whole-slide images could have a size of at least 4.000 × 4.000 pixels, or at least 10.000 × 10.000 pixels, or at least 1E6 × 1E6 pixels.

In particular, if the first medical image is a histopathology slide image, also each training medical image, the of the plurality of second medical images and/or the similar medical image are histopathology slide image. In particular, if the first medical image is a whole-slide image, also each training medical image, the of the plurality of second medical images and/or the similar medical image are whole-slide images.

The inventors recognized that histopathology slide images or whole-slide images can depict detailed morphological structures of cells being subject to certain diseases or other non-patient data. Based on those morphological structures it is possible to provide very relevant similar medical images based on certain morphological structures indicative of certain diseases or other non-patient data.

According to a further aspect of the invention the non-imaging data comprises at least one of the following categories: omics data, diagnosis data, treatment history, and/or demographic data.

In general, the term "omics data" refers to analysis results related to biological molecules of a patient, e.g., generated by various 'omics' technologies, such as genomics, transcriptomics, proteomics, and metabolomics. Biological molecules encompass genes, RNA transcripts, proteins, and/or metabolites. Omics data can provide comprehensive insights into the functioning and interactions within biological systems. The inventors have recognized that omics data can reveal certain details about the biological expression of a patient that can be advantageously used for determining feature vectors suitable for finding similar medical images. In particular, omics data describes the functional makeup of the diseases like cancer, so that retrieving potions with similar omics structure has a particular diagnostic value.

In general, the term "diagnosis data" refers to data related to the identification and classification of diseases, disorders, ad/or medical conditions in patients. It encompasses various types of data, including medical records, diagnostic test results, patient symptoms, and clinical observations. The inventors have recognized data diagnosis data gives detailed and curated information about a patient and/or a certain disease of a patient, so that diagnosis data can be advantageously used for determining feature vectors suitable for finding similar medical images.

In general, the term "treatment history" refers to a record of at least a subset of the medical interventions and therapies that a patient has undergone for a particular disease, condition, or ailment. Treatment history data can comprise information such as the types of treatments administered, dosages, durations, treatment outcomes, and any adverse reactions or side effects experienced. The inventors have recognized that treatment history yields valuable information about a certain disease of a patient and its reaction to certain treatment options. Treatment data can be advantageously used for determining feature vectors suitable for finding similar medical images, and also give hints for successful and non-successful therapies for similar patients.

In general, the term "demographic data" refers to a set of characteristics and information about a patient that provides insights into its personal background, e.g., social, cultural, and economic background. Demographic data can include factors such as age, gender, race and/or ethnicity, but also geographical location, education level, income, and occupation. The inventors recognized that demographic data can be indicative for the similarity of certain patients and can be advantageously used for determining feature vectors suitable for finding similar medical images.

According to a further aspect of the invention the non-imaging data comprises omics data, and the omics data comprises at least on of genomic data, transcriptomic data, proteomic data, and metabolomic data.

In general, the term "genomic data" refers to a set of genetic information or at least parts of the DNA (acronym for "deoxyribonucleic acid") sequence of a patient. It can comprise at least parts of the genome of a patient, including the arrangement and composition of genes, variations, and mutations within the DNA. The inventors recognized that genomic data is indicative of a patient's inherited traits, susceptibility to diseases, and response to medications, and thus can be advantageously used for determining feature vectors suitable for finding similar medical images.

In general, the term "transcriptomic data" refers to a set of information regarding the expression of genes in a specific tissue or cell type of a patient. It can comprise analysis results of RNA molecules that are transcribed from genes, providing insights into which genes are active and producing proteins in a given biological sample of the patient. The inventors recognized that transcriptomic data is indicative of which genetic information of a data is in fact expressed in a certain patient, and thus can be advantageously used for determining feature vectors suitable for finding similar medical images.

In general, the term "proteomic data" refers to a set of information regarding the proteins present in a specific tissue, cell, or biological sample of a patient. It can comprise the types, quantities, modifications, and interactions of proteins within the biological sample of a patient. The inventors recognized that proteomic data provides valuable insights into the functional aspects of proteins, their roles in cellular processes, and their involvement in diseases, and thus can be advantageously used for determining feature vectors suitable for finding similar medical images.

In general, the term "metabolomic data" refers to information related to molecules (known as metabolites) present in a patient's biological sample such as blood, urine, or tissue. It can comprise the identification and quantification of metabolites and/or the overall metabolic profile of a patient. The inventors recognized that metabolomic data provides valuable information about the metabolic state, disease markers, and response to therapies related to a patient, and thus can be advantageously used for determining feature vectors suitable for finding similar medical images.

According to a further possible aspect of the invention the non-imaging data comprises omics data and the training medical image displays a tumor of the patient, and wherein the omics data relates to the type of a cancer of the patient.

The inventors recognized that omics data can give detailed information about specific properties of a cancer of the patient which can be used to determine feature vectors suitable for finding similar medical images related to the same patient types.

According to a further possible aspect of the invention the training medical image is a histopathology slide image displaying tumor cells of the patient, particularly a whole-slide image displaying tumor cells of the patient.

The inventors recognized that histopathology slide images displaying a tumor of the patient comprise detailed information about the morphology of the tumor cells which can be used for determining feature vectors suitable for finding similar medical images.

According to a further aspect of the invention the first machine learning model was trained by training a second machine learning model comprising the first machine learning model, wherein the second machine learning model was trained to predict non-imaging data based on medical images, wherein advantageously the second machine learning model is a classifier.

According to a further possible aspect of the invention, the second machine learning model comprises the first machine learning model and a classification unit, wherein the classification unit processes the output of the first machine learning model and determines a classification value. In particular, the classification unit comprises or consist of additional neural network layers.

According to a further possible aspect of the invention, at least one parameter of the second machine learning network is based on a difference between the output of the second machine learning network when applying the second machine learning network to a training medical image and non-imaging data corresponding to the training medical image medical image.

According to a further aspect of the invention by applying the first machine learning network to a medical image output data is generated, the output data comprising a feature vector and non-imaging data. In particular, the feature vector and the non-imaging data are different.

The inventors recognized that by using a first machine learning network outputting both non-imaging data and the feature vector the first machine learning network can be used both for determining similar images and determining respective non-imaging data.

According to a further aspect of the invention, the first machine learning model comprises a feature unit and an attention unit, wherein the attention unit is based on an attention mechanism. According to this aspect, the step of determining the first feature vector comprises determining a plurality of sub-features by applying the feature unit to the first medical image, and determining the first feature vector by applying the attention unit to the plurality of sub-features.

In particular, each of the sub-features can be a vector having the same dimensionality as the first feature vector.

In general, attention mechanism are sub-units of machine learning models that allow the machine learning model to focus on specific parts of the input of the attention mechanism by assigning different weights to different parts of the input attention mechanism. This weighting is typically based on the relevance of each part of the input to the task to be executed by the machine learning model. In neural networks, attention mechanism can correspond to at least one network layer. In particular, attention mechanisms can refer to self-attention, dot-product attention, scaled dot-product attention, multi-head attention and/or structured attention.

The inventors recognized that by using an attention mechanism within the machine learning model the accuracy of the predictions of the machine learning model is improved by allowing the machine learning model to pay attention to the most relevant information. Furthermore, an attention mechanism can make the machine learning model more efficient by only processing the most important data, reducing the computational resources required and making the machine learning model more scalable. Furthermore, the attention weights learned by the model can provide insight into which parts of the data are the most important, which can help improve the machine learning model's interpretability.

According to a further aspect of the invention the step of determining the first feature vector comprises reducing dimensionality of the sub-features by principal component analysis and/or applying learnable bottleneck layers.

In general, principal component analysis is an orthogonal linear transformation that transforms input data to a new coordinate system such that the greatest variance by some scalar projections of the data is mapped to the first coordinate (called the first principal component), the second greatest variance on the second coordinate, and so forth. Based on a principal component analysis, the dimensionality can be reduced by only keeping a certain number of the first coordinates. In general, a learnable bottleneck layer is a layer of a neural network comprising fewer output nodes than input nodes.

The inventors recognized that by using dimensionality reduction techniques for the feature vectors storing feature vectors (in particular related to the plurality of second medical image) requires less memory capacity, and comparing feature vectors requires less computation power. This allows to provide similar medical images faster and/or with higher accuracy.

According to a further aspect of the invention the first machine learning model is a Deep Local Feature Network. A Deep Local Feature Network, as well as its benefits, are described in the article H. Noh et al., "Large-Scale Image Retrieval with Attentive Deep Local Features", arXiv:1612.06321 (2016). This article is incorporated by reference into this disclosure.

According to a further aspect of the invention the method comprises determining a segmentation of a tumor within the first medical image, wherein, in the step of determining a first feature vector, the machine learning model is applied to a part of the first medical image corresponding to the segmented tumor.

In particular, the part of the first medical image corresponding to the tumor can be rectangular or quadratic patch of the image. In particular, the rectangular or quadratic patch can contain all of the segmented parts of the tumor, or the segmentation can contain the while rectangular or quadratic patch. Alternatively, the rectangular or quadratic patch and the segmentation of the tumor can overlap.

The inventors recognized that using parts of the medical image corresponding to a tumor as input for the machine learning model gives more accurate results for similar patient search, because in many situations similar patients should be determined for a cancer disease, and the imaging features relating to the tumor are particularly indicative for the type of cancer disease.

According to a further aspect of the invention the method comprises dividing the first medical image into a plurality of first patches, wherein, in the step of determining the first feature vector, the first machine learning model is applied to one of the first patches. In particular, the first machine learning model can be applied to a plurality of the first patches to determine several first feature vectors. In particular, the first machine learning model can be applied to all of the first patches to determine a first feature vector for all of the first patches.

In particular, the patches can be rectangular or quadratic patches. In particular, the patches can be mutually exclusive (so that for every pixel of the first medical images there is not more than one patch containing this pixel) and/or covering (so that for every pixel of the first medical images there is at least one patch containing this pixel). In particular, all the patches can have the same size in terms of number of pixels.

The inventors recognized that the first medical image often comprises a huge number of pixels (in particular, for whole-slide images) that cannot be processed with a high accuracy by a machine learning model. By dividing the first medical image into patches and applying the machine learning model to the patches the input data of the machine learning model can be reduced and a better computational efficiency can be achieved. Furthermore, by calculating feature vectors for patches it is possible to associated the relevant similar patches in the comparison images.

According to a further possible aspect of the invention for each of the first patches a corresponding first feature vector is determined by applying the first machine learning model to the respective first patch. The inventors recognized that by calculating several first feature vectors for different patches of the first medical image the similar medical image can be determined not based on the whole image, but for potentially more relevant subparts. Furthermore, it would be possible to find several similar medical images for different parts of the first medical image.

According to a further possible aspect of the invention the first feature vector is based on the outputs of the first machine learning model when applied to each first patch from a subset of the first patches. In particular, the first feature vector can be an average or a weighted average of the outputs of the first machine learning model when applied to each first patch from the subset of first patches. The inventors recognized that by calculating the first feature vector based on a plurality of patches the overall impression of the first medical image as well as information from the whole first medical image can be included into the calculation of the first feature vector, where at the same time it is not necessary to process the whole first medical image at the same time, increasing computational efficiency.

According to a further possible aspect of the invention the subset of the first patches is selected based on the attention mechanism of the first machine learning model. The inventors recognized that using the attention mechanism leads to the most relevant first patches to be included as the basis for calculating the first feature vector.

According to a further aspect of the invention the similar medical image comprises a plurality of second patches, wherein one of the second patches is related to the similar feature vector, and providing the similar medical image comprises displaying an association between the one of the first patches and the one of the second patches.

According to a further possible aspect of the invention providing the similar medical image comprises displaying associations between the first patches and the second patches based on a similarity of feature vectors related to the first patches and the second patches. In particular, an association can be displayed if the similarity fulfills a certain threshold.

In general, an association between the one of the first patches and the one of the second patches is a (visual) indication that the one of the first patches and the one of the second patches are related. In particular, an association can be a visual indication marking both the one of the first patches and the one of the second patches in the same way (e.g., with the same border type or with the same symbol). Alternatively or additionally, an association can be a visual indication connecting the one of the first patches and the one of the second patches (e.g., by a lines). Alternatively or additionally, an association can be a visual indication based on the spatial relationship of the one of the first patches and the one of the second patches on a display (e.g., displaying the one of the first patches and the one of the second patches side-by-side).

The inventors recognized that by displaying an association the user can be easily identify related patches in the first medical image and the similar medical image that led to the selection of the similar medical image.

In a second aspect the invention relates to a computer-implemented method for providing a first machine learning model. The method comprises receiving a training dataset comprising a training medical image and related non-imaging data. Furthermore, the method comprises determining output data by applying a second machine learning model to the training medical image, wherein the second machine learning model comprises the first machine learning model. The method furthermore comprises modifying a parameter of the second machine learning model based on a difference between the non-imaging data and the output data. The method furthermore comprises extracting the first machine learning model from the second machine learning model and providing the first machine learning model.

In particular, the difference between the non-imaging data and the output data can be a d-norm or the mean squared error (corresponding to the sum of squared differences of entries in a vector). In particular, modifying a parameter of the second machine learning model can be based on minimizing the difference between the non-imaging data and the output data, e.g., by using a gradient descent method. In particular, if the second machine learning model is a neural network, the backpropagation algorithm can be used within the gradient descent method. In particular, the first machine learning model comprises the parameter of the second machine learning model.

In particular, extracting the first machine learning model from the second machine learning model (in the present case where the second machine learning model comprises the first machine learning model) can correspond to cropping the second machine learning model to comprise only parts also contained in the first machine learning model. If the first and the second machine learning models are artificial neural networks, extracting the first machine learning model from the second machine learning model can correspond to only using nodes and weights of the second machine learning model that are also contained in the first machine learning model.

The inventors recognized that based on the presented method a first machine learning model can be trained for use in one of the methods according to the first aspect of the invention.

According to a third aspect the invention relates to an image providing system for providing a similar medical image, comprising:
- means for receiving a first medical image related to a first patient;
- means for determining a first feature vector by applying a first machine learning model to the first medical image, wherein the first machine learning model was trained based on training datasets, each training dataset comprising a training medical image and related non-imaging data;
- means for receiving a plurality of second feature vectors, wherein the plurality of second feature vectors is the result of applying the first machine learning model to a plurality of second medical images;
- means for determining, based on the first feature vector, a similar feature vector from the plurality of second feature vectors;
- means for selecting the similar medical image from the plurality of second medical images, wherein the similar medical image is related to the similar feature vector; and
- means for providing the similar medical image.

According to a fourth aspect the invention relates to an image providing system for providing a similar medical image, comprising a processor and an interface configured to:
- receiving a first medical image related to a first patient;
- determining a first feature vector by applying a first machine learning model to the first medical image,
   wherein the first machine learning model was trained based on training datasets, each training dataset comprising a training medical image and related non-imaging data;
- receiving a plurality of second feature vectors,
   wherein the plurality of second feature vectors is the result of applying the first machine learning model to a plurality of second medical images;
- determining, based on the first feature vector, a similar feature vector from the plurality of second feature vectors;
- selecting the similar medical image from the plurality of second medical images, wherein the similar medical image is related to the similar feature vector; and
- providing PROV-IMG-S the similar medical image.

In particular, the image providing systems can be configured to execute the method for providing a similar medical image according to the invention and its aspects. The image providing systems are configured to execute the method and its aspects by its means, interface and/or its processor being configured to execute the respective method steps.

According to a fifth aspect the invention relates to a computer program product comprising instructions which, when the program is executed by a computer, cause the computer to carry out the method for providing a similar medical image according to the invention and its aspects.

According to a sixth aspect the invention relates to a computer-readable storage medium comprising instructions which, when executed by a computer, cause the computer to carry out the method for providing a similar medical image according to the invention and its aspects.

The realization of the invention or one of its aspects by a computer program product and/or a computer-readable medium has the advantage that already existing servers, devices and clients can be easily adapted by software updates in order to work as proposed by the invention.

The said computer program products can be, for example, a computer program or comprise another element apart from the computer program. This other element can be hardware, for example a memory device, on which the computer program is stored, a hardware key for using the computer program and the like, and/or software, for example a documentation or a software key for using the computer program.

According to a sevenths aspect the invention relates to a model providing system for providing a first machine learning model, comprising:
- means for receiving a training dataset comprising a training medical image and related non-imaging data,
- means for determining output data by applying a second machine learning model to the training medical image, wherein the second machine learning model comprises the first machine learning model,
- means for modifying a parameter of the second machine learning model based on a difference between the non-imaging data and the output data,
- means for extracting the first machine learning model from the second machine learning model,
- means for providing the first machine learning model.

According to an eights aspect the invention relates to a model providing system for providing a first machine learning model, comprising a processor and an interface configured for:
- receiving a training dataset comprising a training medical image and related non-imaging data,
- determining output data by applying a second machine learning model to the training medical image, wherein the second machine learning model comprises the first machine learning model,
- modifying a parameter of the second machine learning model based on a difference between the non-imaging data and the output data,
- extracting the first machine learning model from the second machine learning model,
- providing the first machine learning model.

In particular, the model providing systems can be configured to execute the method for providing a first machine learning model according to the invention and its aspects. The image providing systems are configured to execute the method and its aspects by its means, interface and/or its processor being configured to execute the respective method steps.

According to a nineth aspect the invention relates to a computer program product comprising instructions which, when the program is executed by a computer, cause the computer to carry out the method for providing a first machine learning model according to the invention and its aspects.

According to a tenth aspect the invention relates to a computer-readable storage medium comprising instructions which, when executed by a computer, cause the computer to carry out the method for providing a first machine learning model according to the invention and its aspects.

The realization of the invention or one of its aspects by a computer program product and/or a computer-readable medium has the advantage that already existing servers, devices and clients can be easily adapted by software updates in order to work as proposed by the invention.

The said computer program products can be, for example, a computer program or comprise another element apart from the computer program. This other element can be hardware, for example a memory device, on which the computer program is stored, a hardware key for using the computer program and the like, and/or software, for example a documentation or a software key for using the computer program.

The properties, features and advantages of this invention described above, as well as the manner they are achieved, become clearer and more understandable in the light of the following description and embodiments, which will be described in detail in the context of the drawings. This following description does not limit the invention on the contained embodiments. Same components or parts can be labeled with the same reference signs in different figures. In general, the figures are not for scale.

The numbering and/or order of method steps is intended to facilitate understanding and should not be construed, unless explicitly stated otherwise, or implicitly clear, to mean that the designated steps have to be performed according to the numbering of their reference signs and/or their order within the figures. In particular, several or even all of the method steps may be performed simultaneously, in an overlapping way or sequentially.

In the following:
- Fig. 1: displays a data flow diagram according to an embodiment of the invention,
- Fig. 2: displays a data flow diagram according to another embodiment of the invention,
- Fig. 3: displays a data flow diagram according to another embodiment of the invention related to the training of the first machine learning model,
- Fig. 4: displays the structure of a first and a second machine learning model according to an embodiment of the invention,
- Fig. 5: displays a flowchart of a method for providing a similar medical image according to an embodiment of the invention,
- Fig. 6: displays a flowchart of a method for providing a similar medical image according to another embodiment of the invention,
- Fig. 7: displays a flowchart of a method for providing a first machine learning model according to an embodiment of the invention,
- Fig. 8: displays an example of a first medical image being a whole-slide histopathology image,
- Fig. 9: displays associations between first patches of a first medical image and second patches of a similar medical image,
- Fig. 10: displays an image providing system according to an embodiment of the invention,
- Fig. 11: displays a model providing system according to an embodiment of the invention.

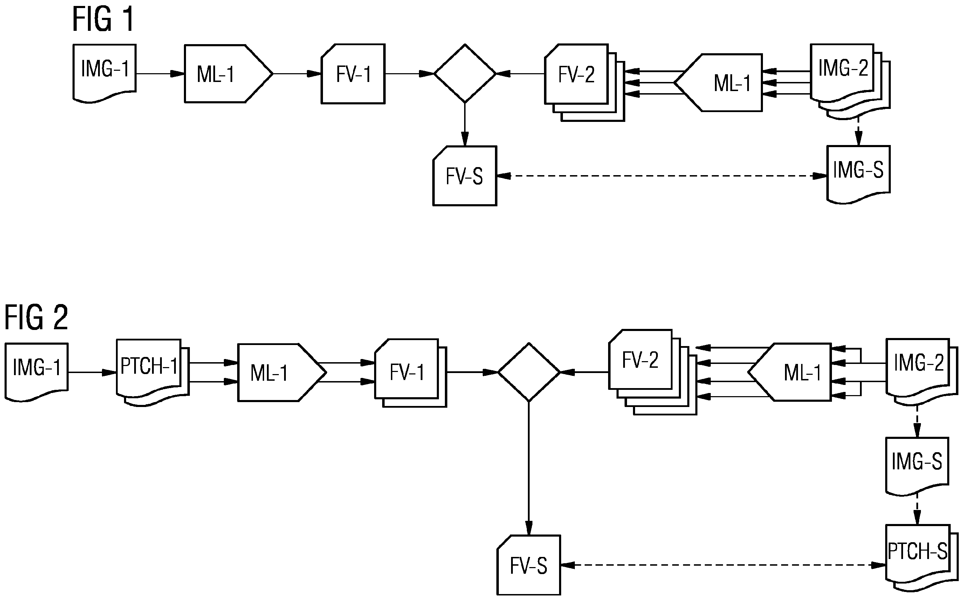

Fig. 1 displays a data flow diagram according to an embodiment of the invention.

In this embodiment, the first medical image IMG-1 is directly used as input to the first machine learning model ML-1, so that a first feature vector FV-1 is generated. Alternatively, it would be possible to use the first medical image IMG-1 and additional input data, e.g., imaging meta-data or patient demographic data. In this embodiment, the first medical image IMG-1 is a user-selected patch of 150×150 pixel extracted from a whole-slide pathology image with H&E stain. The first feature vector FV-1 is a 512-dimensional vector with real entries.

In the same way, each second medical images IMG-2 of the plurality of second medical images IMG-2 is used as input to the first machine learning model ML-1, so that a plurality of second feature vectors FV-2 is generated. Alternatively, it would be possible to use the second medical image IMG-2 and additional input data, e.g., imaging meta-data or patient demographic data. In this embodiment, all the second medical images IMG-2 are patches of 150×150 pixel extracted from whole-slide images, and all the second feature vectors FV-2 are 512-dimensional vectors with real entries.

Furthermore, the first feature vector FV-1 is compared with each second feature vector FV-2 of the plurality of second feature vectors FV-2. In this embodiment, a similarity measure is calculated for every comparison based on the sum of squared differences s (v,wᵢ) = (v - wᵢ)·(v - wᵢ)/512, wherein *"*·*"* represents the scalar product / dot product between two vectors, and wherein v is the first feature vector and wᵢ is one of the second feature vectors FV-2. From all comparisons, the second feature vector FV-2 with the highest similarity (here, with minimal s(v,wᵢ) ≥ 0) is selected as similar feature vector FV-S. Alternatively, instead of the sum of squared differences methods as described in Section 4 of the document Teichmann et al., "Detect-to-Retrieve: Efficient Regional Aggregation for Image Search", arXiv:1812.01584v2 can be used for comparing feature vectors FV-1, FV-2 and sets of feature vectors.

Based on the similar feature vector FV-S, the similar medical image IMG-S can be determined, since the similar medical image IMG-S is the image that was used as input for the first machine learning model ML-1 to calculate the similar feature vector FV-S. For example, there can be a database table associating second feature vectors FV-2 and second medical images IMG-2 that can be queried to determine the similar medical image IMG-S based on the similar feature vector FV-S.

Of course, it is also possible to select several similar feature vectors FV-S and several corresponding similar medical images IMG-S. For example, a predetermined number of second feature vectors FV-2 can be selected as similar feature vectors FV-S (e.g., those with the highest similarity), or all feature vectors FV-2 fulfilling a threshold similarity can be selected as similar feature vectors FV-S.

Fig. 2 displays a data flow diagram according to another embodiment of the invention.

Different from the embodiment displayed in Fig. 1, in this embodiment the first medical image IMG-1 is not used directly as input for the first machine learning model ML-1, but first patches PTCH-1 from the first medical image IMG-1. In this embodiment, the first medical image IMG-1 being a whole-slide image is divided into (possibly overlapping) first patches PTCH-1 of 150×150 pixel. For each of the first patches PTCH-1, by applying the first machine learning model ML-1 a first feature vector FV-1 is determined, wherein first feature vector FV-1 is a 512-dimensional vector with real entries.

Also the plurality of second medical images IMG-2 is divided into patches, and for each patch of each second medical image IMG-2 a second feature vector FV-2 is calculated based on applying the first machine learning model ML-1 to the respective patch.

In this embodiment, for each first feature vector FV-1 a similar feature vector FV-S is determined from the plurality of second feature vectors as described with respect to Fig. 1. Alternatively, it is also possible to determine a similar feature vector FV-S for a certain subset of the first feature vectors FV-1, e.g., those where the respective patches correspond to segmented parts of a tumor within the first medical image IMG-1. Alternatively, it is also possible to determine the pair comprising of one of the first feature vectors FV-1 and one of the second feature vectors FV-2 where the mutual similarity measure is highest, and to pick the determined second feature vector FV-2 as the similar feature vector FV-S.

Based on the at least one similar feature vector FV-S the at least one corresponding similar medical image IMG-S can be selected. It is possible that the number of similar medical images IMG-S is smaller than the number of the at least one similar feature vectors FV-S, if several of these similar feature vectors FV-S correspond to (different second patches PTCH-S of) the same similar medical image IMG-S. Alternatively, it is also possible to select the at least one second patches PTCH-S corresponding to the similar feature vectors FV-S as similar medical images IMG-S (i.e., not retrieve the full similar medical images IMG-S, but only the respective second patches PTCH-S).

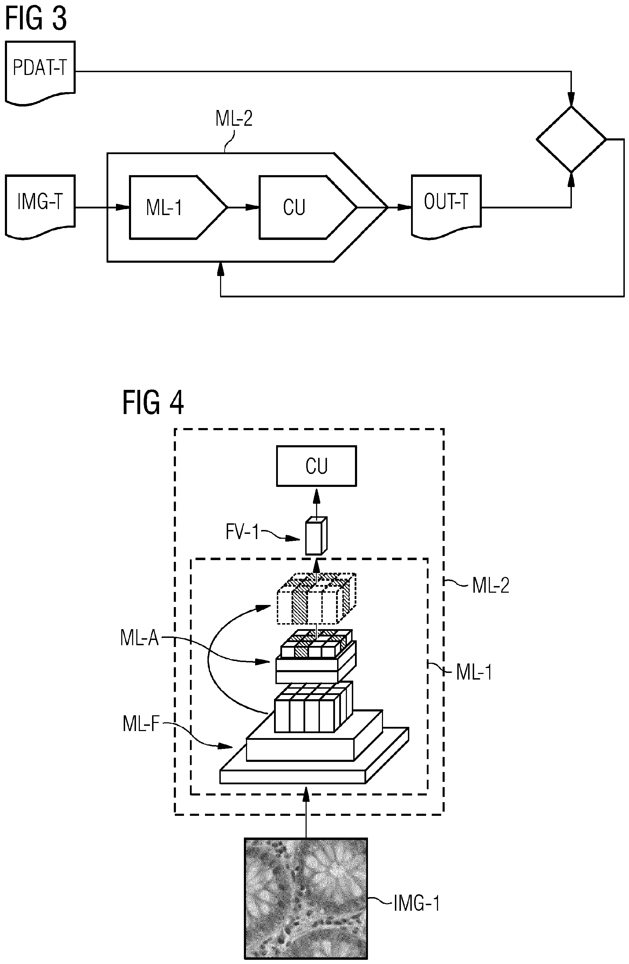

Fig. 3 displays a data flow diagram according to another embodiment of the invention related to the training of the first machine learning model ML-1.

In this embodiment, the first machine learning model ML-1 is a part of a second machine learning model ML-2. In addition to the first machine learning model ML-1 the second machine learning model comprises a classifier unit CU. The second machine learning model ML-2 can also comprise further components. In this embodiment, the second machine learning model ML-2 is a neural network comprising of a plurality of layers, and the first machine learning model ML-1 comprises a first subset of consecutive layers, and the classifier unit CU comprises a second subset of consecutive layers.

During training of the machine learning models ML-1, ML-2, training datasets are used, wherein each training dataset comprises a training medical image IMG-T and related non-imaging data PDAT-T. In particular, the training medical image IMG-T and the non-imaging data PDAT-T are related to the same patient. In this embodiment, the training medical image IMG-T is a user-selected patch of 150×150 pixel extracted from a whole-slide pathology image with H&E stain. However, it would also be possible to use training medical images of other type, e.g., recorded by a computed tomography apparatus or a magnetic resonance imaging apparatus.

The training medical image IMT-T is used as input for the second machine learning model ML-2 and for the first machine learning model ML-1, since in this embodiment the first machine learning model ML-1 is the first part of the second machine learning model ML-2 with respect to the processing direction of a feed forward network. When applied to the training medical image IMT-T the second machine learning model ML-2 creates output data OUT-T. Internally, the first machine learning model ML-1 calculates a feature vector, and the classifier unit CU takes as input the feature vector and calculates as output the output data OUT-T.

Within the training, the output data OUT-T and the non-imaging data PDAT-T are compared, and based on the comparison, at least one parameter of the second machine learning model ML-2 is adapted. In this embodiment, the at least one parameter of the second machine learning model ML-2 is a parameter of the first machine learning model ML-1 and/or a parameter of the classifier unit CU.

In this embodiment, the non-imaging data PDAT-T comprises genomic data and data related to a molecular subtype of colorectal cancer of the patient (in general, one of the classes "CMS1", "CMS2", "CMS3" and "CMS4" for "hypermutated subtype", "canonical subtype", "metabolic subtype" and "mesenchymal subtype"). The genomic data refers to an information about whether the genome of the patient comprises a mutation in the BRAF gene ("proto-oncogene B-Raf"), and specifically, whether the BRAF gene comprises the BRAF V600E mutation. In particular, the non-imaging data PDAT-T can be a three-dimensional vector p, wherein p₁ ∈ {0,1} depending on whether there is a mutation in the BRAF gene of the patient, p₂ ∈ {0,1} depending on whether the genome of the patient comprises the BRAF V600E mutation, and p₃ ∈ {1, 2, 3, 4} depending on the molecular subtype of colorectal cancer of the patient.

In this embodiment, also the output data OUT-T is a three-dimensional vector with real numbers v₁, v₂ ∈ [0,1] and v₃ ∈ [0,4]. Within the training, the parameter of the second machine learning network ML-2 is adapted based on the backpropagation algorithm using the loss function L = (p₁ - v₁)² + (p₂ - v₂)² + (p₃ - v₃)², which is based on the difference of the non-imaging data PDAT-T and the output data OUT-T.

Fig. 4 displays a first machine learning model ML-1 and a second machine learning model ML-2 for use in one of the embodiments of the invention.

In this embodiment, the second machine learning model ML-2 comprises the first machine learning model ML-1 and a classifier unit CU, wherein the first machine learning model ML-1 takes as input a first medical image IMG-1 and creates as an output a first feature vector FV-1, and the classifier unit CU takes as input the first feature vector FV-1 and creates output data OUT-T corresponding to non-imaging data PDAT-T.

In this embodiment, the input images IMG-1 are square patches of size 250x250 pixel selected from stained whole-slide images. The first machine learning model ML-1 comprises a feature unit ML-F and an attention unit ML-A, wherein the attention unit ML-A is based on an attention mechanism. The feature unit ML-F takes as input the first medical image IMG-1 and creates as output a plurality of sub-features. The attention unit ML-A takes as input the plurality of sub-features and calculates an attention score for each of the sub-features.

In this embodiment, the feature unit ML-F is derived from the well-known ResNet50 model (e.g., described in He et al., "Deep Residual Learning for Image Recognition", arXiv:1512.03385. However, since the feature unit ML-F should not generate a classification value, but a certain number of feature vectors, a certain number of the last layers of the ResNet50 model are skipped, so that a plurality of sub-feature is generated. For example, there could be 49 sub-features (denoted by fₙ), each being a 512-dimensional vector.

In this embodiment, the attention unit ML-A comprises an attention score function α(fₙ, θ) with learnable parameters θ. The output of the attention unit ML-A is then the weighted sum Σₙ α(fₙ, θ) fₙ, and the parameters θ can be learned based on backpropagation if comparing the output of the classifier unit CU with the ground truth being the non-imaging data PDAT-T.

In this embodiment, the classifier unit CU is a fully connected network taking as input 512 numerical values (corresponding to the entries of the first feature vector FV-1). In other words, the input layer of the classifier unit CU has 512 nodes. The number of output values corresponds to the items of classification that should be calculated by the second machine learning model ML-2. For example, if the non-imaging data PDAT-T is a three-dimensional vector, the output comprises at least three (preferably exactly three) numerical values, and the output layer of the classifier unit CU comprises 3 nodes. In this situation, there can be three hidden layers with 120, 40 and 15 nodes. Alternatively, the classifier unit can also be a convolutional neural network.

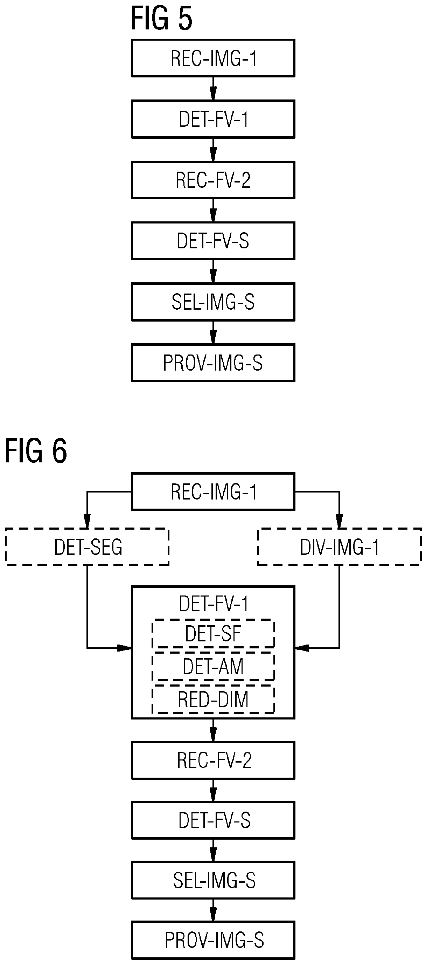

Fig. 5 displays a flowchart according to an embodiment of the method for providing a similar medical image IMG-S.

The first step of the displayed method is receiving REC-IMG-1 a first medical image IMG-1 related to a first patient PAT-1. In this embodiment, the first medical image IMG-1 is a rectangular patch of 250x250 pixels selected from a whole-slide digital pathology image. Alternatively, one could also use other types of first medical images IMG-1. For example, one could use a DICOM image created in a radiological examination with magnetic resonance imaging or computed tomography. The step of receiving REC-IMG-1 is executed by an interface PSYS.IF of an image providing system PSYS.

The next step of the displayed method is determining DET-FV-1 a first feature vector FV-1 by applying a first machine learning model ML-1 to the first medical image IMG-1 by a computation unit PSYS.CU of the image providing system PSYS. The machine learning model ML-1 has a structure as displayed in and explained with respect to Fig. 4. The first machine learning model ML-1 was trained based on training datasets, each training dataset comprises a training medical image IMG-T and related non-imaging data PDAT-T. The training medical images IMG-T are patches of size 250x250 pixels of whole-slide images, in particular, not containing medical images related to the first patient PAT-1. Alternatively, one could use other types of training medical images IMG.T, however, advantageously the size and the type of the training medical images IMG-T should match the size and the type of the first medical image IMG-1. In this embodiment, the non-imaging data PDAT-T has the structure as described with respect to Fig. 3, alternatively, one could also use other non-imaging data PDAT-T. In this embodiment, the training medical image IMG-T and the non-imaging data PDAT-T within each training dataset corresponds to the same patient.

The next step of the embodiment displayed in Fig. 5 is receiving REC-FV-2 a plurality of second feature vectors FV-2 by the interface PSYS.IF of the image providing system PSYS, wherein the plurality of second feature vectors FV-2 is the result of applying the first machine learning model ML-1 to a plurality of second medical images IMG-2. In this embodiment, the second medical images IMG-2 are patches of size 250x250 pixels of whole-slide images. Alternatively, one could use other types of second medical images IMG-2, however, advantageously the size and the type of the second medical images IMG-2 should match the size and the type of the first medical image IMG-1.

In this embodiment, each of the second feature vectors FV-2 has the same structure and the same dimensionality as the first feature vector FV-1. In particular, each of the second feature vectors FV-2 is a 512-dimensional vector with real entries. In this embodiment, each of the second feature vectors FV-2 is the result of applying the first machine learning model ML-1 to exactly one of the second medical images IMG-2, in other words, there is a bijective map between the second medical images IMG-2 and the second feature vectors FV-2.

The next step of this embodiment is determining DET-FV-S, based on the first feature vector FV-1 and with the computation unit PSYS.CU of the image providing system, a similar feature vector FV-S from the plurality of second feature vectors FV-2. The similar feature vector FV-S is one of the second feature vectors FV-2. In particular, the similar feature vector FV-2 is the one of the second feature vectors FV-2 that is closest to the first feature vector FV-1 according to a pre-defined metric. In this embodiment, the Euclidean distance is used as metric, so that the similar feature vector FV-S is the one of the second feature vectors FV-2 with the smallest Euclidean distance to the first feature vector FV-1. Alternatively, other metrics could be used like the Manhattan metric or Cosine similarity.

The next step of the method displayed in Fig. 5 is selecting SEL the similar medical image IMG-S from the plurality of second medical images IMG-2, wherein the similar medical image IMG-S is related to the similar feature vector FV-S. In this embodiment, the similar medical image IMG-S is related to the similar feature vector FV-S by the similar feature vector FV-S having been calculated by applying the first machine learning model ML-1 to the similar medical image IMG-S. In particular, the relation between the second medical images IMG-2 and the second feature vectors FV-2 can be stored in a database, so that selecting SEL the similar medical image IMG-S can comprise accessing or querying this database to find the similar medical image IMG-S related to the similar feature vector FV-S.

The last step of the displayed embodiment is providing PROV-IMG-S the similar medical image IMG-S with the interface PSYS.IF of the image providing system PSYS. In particular, the step of providing PROV-IMG-S the similar medical image IMG-S can comprise transmitting the similar medical image IMG-S to another entity, storing the similar medical image IMG-S in a storage unit and/or displaying the similar medical image IMG-S on a display unit.

Fig. 5 displays a flowchart according to another embodiment of the method for providing a similar medical image IMG-S. In this embodiment, the steps of receiving REC-IMG-1 the first medical image IMG-1, receiving REC-FV-2 the plurality of second feature vectors, determining DET-FV-S the similar feature vector FV-S, selecting SEL-IMG-S the similar medical image IMG-S and providing PROV-IMG-S the similar medical image IMG-S can comprise the same advantageous features and options as described with respect to the embodiment of Fig. 5.

In this embodiment, the method furthermore comprises the optional step of determining DET-SEG a segmentation of a tumor within the first medical image IMG-1 by the computation unit PSYS.CU of the image providing system PSYS. The segmentation can be determined in various ways, e.g., one can use a neural network having a U-Net structure taking as input the first medical image IMG-1 and calculating as output a binary mask of the same size, wherein pixels with value 1 correspond to tumor pixels, and pixels with value 0 correspond to non-tumor pixels. Alternatively, it is also possible to use other segmentation methods that are not based on learning algorithms.

In this embodiment, the method furthermore comprises the optional step of dividing DIV-IMG-1 the first medical image IMG-1 into a plurality of first patches PTCH-1 by the computation unit PSYS.CU of the image providing system PSYS. In this embodiment, the maximal number of non-overlapping first patches PTCH-1 of a predefined pixel size (e.g., 250x250 pixels) is determined. Alternatively, a predefined number of M•N (with M, N being predefined integer numbers) first patches PTCH-1 can be created by equally dividing the first image IMG-1 M times along the first axis and N times along the second axis.

The step of optional step of determining DET-SEG the segmentation of the tumor can be executed before the step of dividing DIV-IMG-1 the first medical image IMG-1 or vice versa. Alternatively, both steps can be executed in parallel.

In this embodiment, within the step of determining DET-FV-1 the first feature vector FV-1 the first machine learning model ML-1 is applied to at least one of the first patches PTCH-1 which corresponds to the segmented tumor (i.e., by having an overlap with the segmented tumor mask). In particular, the first machine learning model ML-1 is applied to all of the first patches PTCH-1 corresponding to the segmented tumor, and for all of these first patches PTCH-1 a separate first feature vector FV-1 is calculated. In particular, this means that the step of determining DET-FV-1 the first feature vector is executed for each of the relevant first patches PTCH-1, and that a similar feature vector FV-S and a similar medical image IMG-S are determine for each of the relevant first patches PTCH-S. Alternatively, one could determine just a single similar feature vector FV-S and a single similar medical image IMG-S based on the feature vector from the plurality of second feature vectors FV-2 that has the largest similarity with any of the first feature vectors FV-1.

Furthermore, in this embodiment the step of determining DET-FV-1 the first feature vector FV-1 comprises the optional subset of determining DET-SF a plurality of sub-features by applying the feature unit ML-F to the first medical image IMG-1, and of determining DET-AM the first feature vector FV-1 by applying the attention unit ML-A to the plurality of sub-features, as explained with respect to Fig. 4.

Furthermore, the step of DET-FV-1 the first feature vector FV-1 comprises the optional subset of reducing RED-DIM dimensionality of the sub-features by principal component analysis and/or applying learnable bottleneck layers.

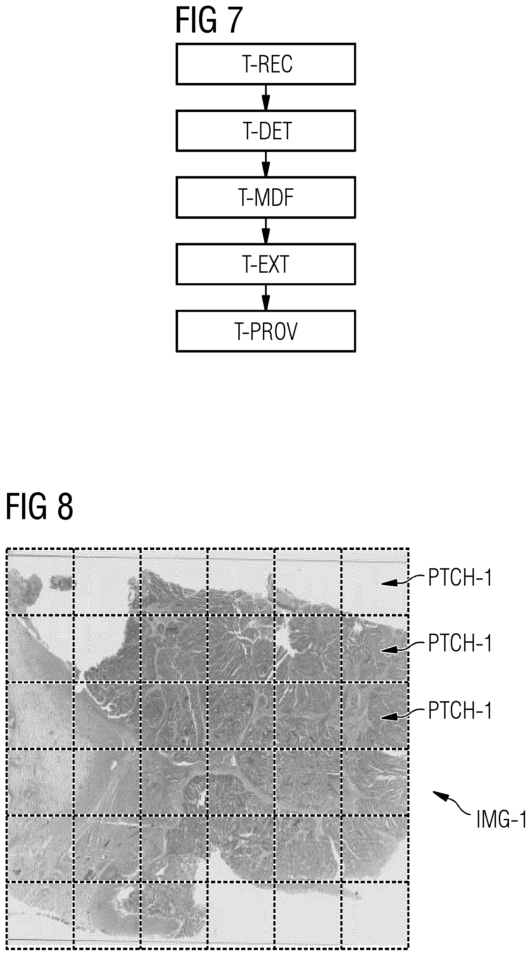

Fig. 7 displays a flowchart of a method for providing a first machine learning model ML-1 according to an embodiment of the invention.

According to the embodiment, the method comprises as a first step receiving T-REC a training dataset comprising a training medical image IMG-T and related non-imaging data PDAT-T. In particular, the training medical image IMG-T and the non-imaging data PDAT-T are related by being acquired from the same patient. The step of receiving T-REC the training dataset is executed by an interface TSYS.IF of a model providing system TSYS.

The next step of the displayed embodiment is determining T-DET output data by applying a second machine learning model ML-2 to the training medical image IMG-T, wherein the second machine learning model ML-2 comprises the first machine learning model ML-1. In particular, the first machine learning model ML-1 and the second machine learning model ML-2 have the structure as described with respect to Fig. 4, so that the second machine learning model ML-2 comprises the first machine learning model ML-1 and a classifier unit CU. The step of determining T-DET output data is executed by a computation unit TSYS.CU of the model providing system TSYS.

The next step of the displayed embodiment is modifying T-MDF a parameter of the second machine learning model ML-2 based on a difference between the non-imaging data PDAT-T and the output data. In particular, the parameter of the second machine learning model ML-2 corresponds to a weight of an edge within a neural network corresponding to the second machine learning model ML-2. In particular, the parameter of the second machine learning model ML-2 is at the same time also a parameter of the first machine learning model ML-1. In particular, the parameter is modifies based on a gradient descend method, in particular, on the backpropagation algorithm. The step of modifying T-MDF the parameter of the second machine learning model ML-2 is executed by the computation unit TSYS.CU of the model providing system TSYS.

The next step of the displayed embodiment is extracting T-EXT the first machine learning model ML-1 from the second machine learning model ML-2. This step is executed by the computation unit TSYS.CU of the model providing system TSYS. In this embodiment, the first machine learning model ML-1 is a subpart of the second machine learning model ML-2, and extracting T-EXT the first machine learning model ML-1 can correspond to deleting all parts that are related to the second machine learning model ML-2 but not to the first machine learning model ML-1.

The last step of the displayed embodiment is providing T-PROV the first machine learning model ML-1, this step is executed by the interface TSYS.IF of the model providing system TSYS.

The steps as described above correspond to a single step of training the first and the second machine learning model ML-1, ML-2. In order to improve the performance of the first machine learning model ML-1, these steps should be executed several times for different training datasets comprising training medical images IMG-T and related non-imaging data PDAT-T.

In this embodiment, a set of 1000 training datasets related to 1000 different patients was used. For each of the training dataset, the non-imaging data PDAT-T is a three-dimensional vector p, wherein p₁ 6 {0,1} depending on whether there is a mutation in the BRAF gene of the patient, p₂ 6 {0,1} depending on whether the genome of the patient comprises the BRAF V600E mutation, and p₃ 6 {1, 2, 3, 4} depending on the molecular subtype of colorectal cancer of the patient. All of this value can be determined by determining a gene panel for the respective patient. Furthermore, for each of the training datasets, the training medical image IMG-T a user-selected patch of 150×150 pixel extracted from a whole-slide pathology image with H&E stain of the respective patient. It would also be possible to select multiple patches from a single whole-slide pathology image in order to increase the number of training datasets.

Fig. 8 displays an example of a first medical image IMG-1 being a two-dimensional stained whole-slide image with N × N pixels. In this embodiment, the first medical image IMG-1 is divided into 36 quadratic first patches PTCH-1, every one of the first patches PTCH-1 being a two-dimensional pixel image with N/6 × N/6 pixels.

The first medical image IMG-1 is based on a tissue sample obtained from a patient by means of biopsy. For the preparation of a tissue slide from the tissue sample a section (also called a 'punch biopsy' or a 'block') was cut out from the tissue sample with a punch tool, with the section being cut into micrometer-thick slices, the tissue slides. The preparation of the tissue slide further comprised the staining of the tissue slide with a H&E (acronym for "hematoxylin and eosin") staining.

For creation of first medical image IMG-1 being a whole-slide images the tissue slide stained with the H&E staining were scanned with a dedicated histopathology scanner, which scans the entire tissue slide mounted on the object carrier and converts it into a color pixel image. The first medical image IMG-1 being a whole-slide images can subsequently be digitally processed and, in particular, be archived in a suitable database.

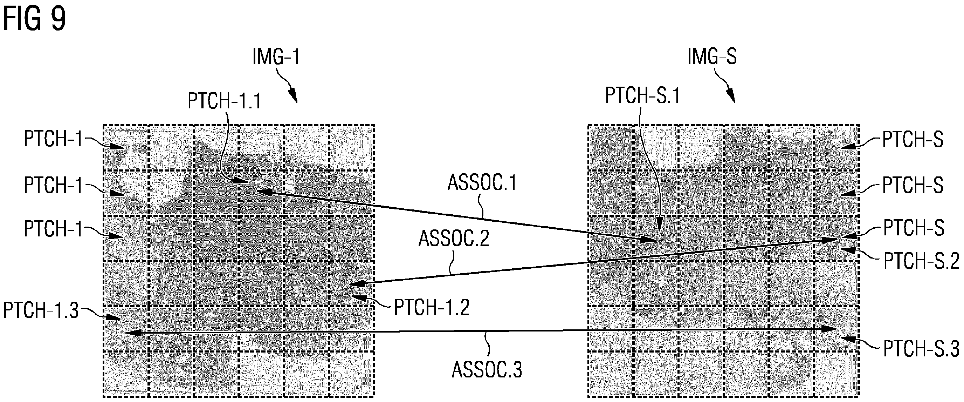

Fig. 9 displays an example of a first medical image IMG-1 and a respective similar medical image IMG-S as determined by one of the methods according to an embodiment of the invention.

In this embodiment, the first medical image IMG-1 comprises a plurality of first patches PTCH-1, and the similar medical image IMG-S comprises a plurality of second patches PTCH-S, wherein each of the patches are two-dimensional and comprise the same number of pixels.

The similar medical image IMG-S was selected from a plurality of second medical images IMG-2, wherein each of the second medical images IMG-2 is a whole-slide image of a tissue sample created by the method as explained with respect to Fig. 8. The selection of the similar medical image IMG-S was based on the similarity of first feature vectors FV-1 determined based on the patches of the first medical image IMG-1 and second feature vectors FV-2 determined based on patches of the second medical images IMG-2.

Furthermore, in this embodiment associations ASSOC.1, ASSOC.2, ASSOC.3 between patches PTCH-1.1, PTCH-1.2, PTCH-1.3 of the first patches PTCH-1 and patches PTCH-S.1, PTCH-S.2, PTCH-S.3 of the second patches PTCH-S are displayed. In this embodiment, the association is displayed by displaying an arrow connecting the respective patch PTCH-1.1, PTCH-1.2, PTCH-1.3 of the first patches PTCH-1 and the respective patch PTCH-S.1, PTCH-S.2, PTCH-S.3 of the second patches PTCH-S.

In particular, in this embodiment the similar medical image IMG-S was selected from the plurality of the second medical images IMG-2 based on the highest similarity of first feature vector FV-1 related to a first patch PTCH-1.1 of the plurality of first patches PTCH-1 and of similar feature vector FV.S related to a first patch PTCH-S.1 of the plurality of second patches PTCH-2. As a result, an association ASSOC.1 is displayed between the first patch PTCH-1.1 of the plurality of first patches PTCH-1 and the first patch PTCH-S.1 of the plurality of second patches PTCH-2.

Furthermore, in this embodiment additional associations ASSOC.2, ASSOC.3 are displayed. These additional associations ASOOC.2, ASSOC.3 correspond to pairs consisting of a patch PTCH-1.2, PTCH-1.3 of the plurality of first patches PTCH-1 and a patch PTCH-S.2, PTCH-S.3 of the plurality of second patches PTCH-2 where the similarity between the related feature vectors is higher than a predefined threshold.

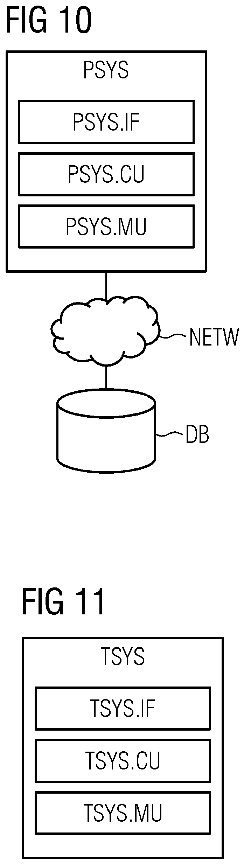

Fig. 10 displays an embodiment of an image providing system PSYS, Fig. 11 displays an embodiment of a model providing system TSYS. The image providing system PSYS is configured for executing a method for providing a similar medical image IMG-S according to embodiments of the invention. The model providing system TSYS is configured for executing a method for providing a first machine learning model ML-1 according to embodiments of the invention.

The image providing system PSYS and/or the model providing system TSYS can be a (personal) computer, a workstation, a virtual machine running on host hardware, a microcontroller, or an integrated circuit. In particular, the image providing system PSYS and/or the model providing system TSYS can be mobile devices, e.g. a smartphone or a tablet. As an alternative, the image providing system PSYS and/or the model providing system TSYS can be a real or a virtual group of computers (the technical term for a real group of computers is "cluster", the technical term for a virtual group of computers is "cloud").

Each of the image providing system PSYS and/or the model providing system TSYS can comprise an interface PSYS.IF, TSYS.IF, a computation unit PSYS.CU, TSYS.CU and a memory unit PSYS.CU, TSYS.CU. An interface PSYS.IF, TSYS.IF can be a hardware interface or as a software interface (e.g. PCIBus, USB or Firewire). A computation unit PSYS.CU, TSYS.CU can comprise hardware elements and software elements, for example a microprocessor, a CPU (acronym for "central processing unit"), a GPU (acronym for "graphical processing unit"), a field programmable gate array (an acronym is "FPGA") or an ASIC (acronym for "application-specific integrated circuit"). A computation unit PSYS.CU, TSYS.CU can be configured for multithreading, i.e., the computation unit can host different computation processes at the same time, executing the either in parallel or switching between active and passive computation processes. Each of the interfaces PSYS.IF, TSYS.IF, the computation units PSYS.CU, TSYS.CU and the memory units PSYS.CU, TSYS.CU can comprise several subunits which are configured to executed different tasks and/or which are spatially separated.

The image providing system PSYS is connected to a database DB via a network NETW. In particular, the database DB stores the plurality of second feature vectors FV-2 and/or the plurality of second medical images IMG-2. The network NETW can be realized as a LAN (acronym for "local area network"), in particular a WiFi network, or any other local connection. Alternatively, the network NETW can be the internet. In particular, the network NETW could be realized as a VPN (acronym for "virtual private network"). Alternatively, the database DB can also be integrated into the image providing system PSYS, e.g., the database DB could be stored within the memory unit PSYS.MU of the image providing system PSYS. In this case the database is connected with an internal connection.

The following clauses are also part of the disclosure:
Clause 1.1: A computer-implemented method for providing a similar medical image IMG-S, comprising:
   - receiving REC-IMG-1 a first medical image IMG-1 related to a first patient PAT-1;
   - determining DET-FV-1 a first feature vector FV-1 by applying a first machine learning model ML-1 to the first medical image IMG-1, wherein the first machine learning model ML-1 was trained based on training datasets, each training dataset comprising a training medical image IMG-T and related non-imaging data PDAT-T;
   - receiving REC-FV-2 a plurality of second feature vectors FV-2,
      wherein the plurality of second feature vectors FV-2 is the result of applying the first machine learning model ML-1 to a plurality of second medical images IMG-2;
   - determining DET-FV-S, based on the first feature vector FV-1, a similar feature vector FV-S from the plurality of second feature vectors FV-2;
   - selecting SEL-IMG-S the similar medical image IMG-S from the plurality of second medical images IMG-2, wherein the similar medical image IMG-S is related to the similar feature vector FV-S; and
   - providing PROV-IMG-S the similar medical image IMG-S.
Clause 1.2: The method of clause 1.1, wherein each second feature vector FV-2 of the plurality of second feature vectors FV-2 is the result of applying the first machine learning model ML-1 to exactly one second medical image IMG-2 of the plurality of second medical images IMG-2.
Clause 1.3: The method of clause 1.2, wherein there is a one-to-one correspondence between the plurality of second feature vectors FV-2 and the plurality of second medical images IMG-2.
Clause 1.4: The method of one of clauses 1.1 to 1.3, wherein the non-imaging data PDAT-T has a different structure than the first feature vector FV-1.
Clause 1.5: The method of clause 1.4, wherein the non-imaging data PDAT-T has a lower dimensionality than the first feature vector FV-1.
Clause 1.6: The method of one of clauses 1.1 to 1.5, wherein the first medical image IMG-1 is a histopathology slide image, particularly a whole-slide image.
Clause 1.7: The method of one of clauses 1.1 to 1.6, wherein the non-imaging data PDAT-T comprises at least one of the following categories: omics data, diagnosis data, treatment history, and demographic data.
Clause 1.8: The method of clause 1.7, wherein the non-imaging data PDAT-T comprises omics data, wherein the omics data comprises at least on of genomic data, transcriptomic data, proteomic data, and metabolomic data.
Clause 1.9: The method of one of clauses 1.6 to 1.8, wherein the non-imaging data comprises omics data, wherein the training medical image IMG-T displays a tumor of the patient, and wherein the omics data relates to the type of a cancer of the patient.
Clause 1.10: The method of clause 1.9, wherein the training medical image is a histopathology slide image displaying tumor cells of the patient, particularly a whole-slide image displaying tumor cells of the patient.
Clause 1.11: The method of one of clauses 1.1 to 1.10, wherein the first machine learning model ML-1 was trained by training a second machine learning model ML-2 comprising the first machine learning model ML-1, wherein the second machine learning model ML-2 was trained to predict non-imaging data PDAT-T based on medical images IMG-T.
Clause 1.12: The method of clause 1.11, wherein the second machine learning model ML-2 is a classifier.
Clause 1.13: The method of clause 1.11 or 1.12, wherein the second machine learning model ML-2 comprises the first machine learning model ML-1 and a classification unit CU, wherein the classification unit CU processes the output of the first machine learning model ML-1 and determines a classification value.
Clause 1.14: The method of clause 1.13, wherein the classification unit CU comprises or consist of additional neural network layers.
Clause 1.15: The method of one of clauses 1.11 to 1.14, wherein at least one parameter of the second machine learning network ML-2 is based on a difference between the output of the second machine learning network ML-2 when applying the second machine learning network to a training medical image and non-imaging data corresponding to the training medical image medical image.
Clause 1.16: The method of one of clauses 1.1 to 1.10, wherein by applying the first machine learning network ML-1 to a medical image IMG-1, IMG-2, IMG-T output data is generated, the output data comprising a feature vector FV-1, FV-2 and non-imaging data PDAT.
Clause 1.17: The method of one of the clauses 1.1 to 1.17, wherein the first machine learning model ML-1 comprises a feature unit ML-F and an attention unit ML-A, wherein the attention unit ML-A is based on an attention mechanism, wherein the step of determining DET-FV-1 the first feature vector FV-1 comprises:
   -- determining DET-SF a plurality of sub-features by applying the feature unit ML-F to the first medical image IMG-1, and
   -- determining DET-AM the first feature vector FV-1 by applying the attention unit ML-A to the plurality of sub-features.
Clause 1.18: The method of clause 1.17, wherein the step of determining DET-FV-1 the first feature vector FV-1 comprises:
   - reducing RED-DIM dimensionality of the sub-features by principal component analysis and/or applying learnable bottleneck layers.
Clause 1.19: The method of clause 1.17 or clause 1.18, wherein the first machine learning model ML-1 is a Deep Local Feature Network.
Clause 1.20: The method of one of clauses 1.1 to 1.19, furthermore comprising:
   - determining DET-SEG a segmentation of a tumor within the first medical image IMG-1,
   wherein, in the step of determining DET-FV-1 a first feature vector FV-1, the machine learning model is applied to a part of the first medical image IMG-1 corresponding to the segmented tumor.
Clause 1.21: The method of one of clauses 1.1 to 1.19, comprising:
   - dividing DIV-IMG the first medical image IMG-1 into a plurality of first patches PTCH-1,
   wherein, in the step of determining DET-FV-1 the first feature vector FV-1, the first machine learning model ML-1 is applied to one of the first patches PTCH-1.
Clause 1.22: The method of clause 1.21, wherein for each of the first patches PTCH-1 a corresponding first feature vector FV-1 is determined by applying the first machine learning model ML-1 to the respective first patch PTCH-1.
Clause 1.23: The method of clause 1.21, wherein the first feature vector FV-1 is based on the outputs of the first machine learning model ML-1 when applied to each first patch from a subset of the first patches PTCH-1.
Clause 1.24: The method of clause 1.23, wherein the subset of the first patches PTCH-1 is selected based on the attention mechanism of the first machine learning model ML-1.
Clause 1.25: The method of clause 1.21 or 1.22, wherein the similar medical image IMG-S comprises a plurality of second patches PTCH-S, wherein one of the second patches PTCH-S is related to the similar feature vector FV-S, and wherein providing PROV-IMG-S the similar medical image IMG-S comprises displaying an association between the one of the first patches PTCH-1 and the one of the second patches PTCH-S.
Clause 1.26: The method of clause 1.25, wherein providing PROV-IMG-S the similar medical image IMG-S comprises displaying associations between the first patches PTCH-1 and the second patches PTCH-S based on a similarity of feature vectors FV-1, FV-S related to the first patches PTCH-1 and the second patches PTCH-S.
Clause 2.1: A computer-implemented method for providing a first machine learning model ML-1, comprising:
   - receiving T-REC a training dataset comprising a training medical image IMG-T and related non-imaging data PDAT-T,
   - determining T-DET output data by applying a second machine learning model ML-2 to the training medical image IMG-T, wherein the second machine learning model ML-2 comprises the first machine learning model ML-1,
   - modifying T-MDF a parameter of the second machine learning model ML-2 based on a difference between the non-imaging data PDAT-T and the output data,
   - extracting T-EXT the first machine learning model ML-1 from the second machine learning model ML-2,
   - providing T-PROV the first machine learning model ML-1.
Clause 2.2: The method according to clause 2.1, wherein the first machine learning model ML-1 comprises the parameter of the second machine learning model ML-2.
Clause 2.3: The method according to clause 2.1 or clause 2.2, wherein modifying the parameter of the second machine learning model ML-2 is based on a gradient descent method, in particular, based on backpropagation.
Clause 3.1: An image providing system PSYS for providing a similar medical image IMG-S, comprising
   - means for receiving REC a first medical image IMG-1 related to a first patient PAT-1;
   - means for determining DET-FV-1 a first feature vector FV-1 by applying a first machine learning model ML-1 to the first medical image IMG-1,
      wherein the first machine learning model ML-1 was trained based on training datasets, each training dataset comprising a training medical image IMG-T and related non-imaging data PDAT-T;
   - means for receiving REC-FV-2 a plurality of second feature vectors FV-2,
      wherein the plurality of second feature vectors FV-2 is the result of applying the first machine learning model ML-1 to a plurality of second medical images IMG-2;
   - means for determining DET-FV-S, based on the first feature vector FV-1, a similar feature vector FV-S from the plurality of second feature vectors FV-2;
   - means for selecting SEL-IMG-S the similar medical image IMG-S from the plurality of second medical images IMG-2, wherein the similar medical image IMG-S is related to the similar feature vector FV-S; and
   - means for providing PROV-IMG-S the similar medical image IMG-S.
Clause 3.2: An image providing system PSYS for providing a similar medical image IMG-S, comprising a processor PSYS.CU and an interface PSYS.IF configured to:
   - receiving REC a first medical image IMG-1 related to a first patient PAT-1;
   - determining DET-FV-1 a first feature vector FV-1 by applying a first machine learning model ML-1 to the first medical image IMG-1,
      wherein the first machine learning model ML-1 was trained based on training datasets, each training dataset comprising a training medical image IMG-T and related non-imaging data PDAT-T;
   - receiving REC-FV-2 a plurality of second feature vectors FV-2,
      wherein the plurality of second feature vectors FV-2 is the result of applying the first machine learning model ML-1 to a plurality of second medical images IMG-2;
   - determining DET-FV-S, based on the first feature vector FV-1, a similar feature vector FV-S from the plurality of second feature vectors FV-2;
   - selecting SEL-IMG-S the similar medical image IMG-S from the plurality of second medical images IMG-2, wherein the similar medical image IMG-S is related to the similar feature vector FV-S; and
   - providing PROV-IMG-S the similar medical image IMG-S.
Clause 3.3: The image providing system SYS of clause 3.1 or clause 3.2, comprising additional means for executing the method according to one of clauses 1.1 to 1.26, or being configured for executing the method of one of clauses 1.1 to 1.26.
Clause 3.4: A computer program product comprising instructions which, when the program is executed by a computer, cause the computer to carry out the method of one of clauses 1.1 to 1.26.
Clause 3.5. A computer-readable storage medium comprising instructions which, when executed by a computer, cause the computer to carry out the method of one of clauses 1.1 to 1.26.
Clause 4.1: A model providing system TSYS for providing a first machine learning model ML-1, comprising:
   - means for receiving T-REC a training dataset comprising a training medical image IMG-T and related non-imaging data PDAT-T,
   - means for determining T-DET output data by applying a second machine learning model ML-1 to the training medical image IMG-T, wherein the second machine learning model ML-2 comprises the first machine learning model ML-1,
   - means for modifying T-MDF a parameter of the second machine learning model ML-2 based on a difference between the non-imaging data PDAT-T and the output data,
   - means for extracting T-EXT the first machine learning model ML-1 from the second machine learning model ML-2,
   - means for providing T-PROV the first machine learning model ML-1.
Clause 4.2: A model providing system TSYS for providing a first machine learning model ML-1, comprising a processor TSYS.CU and an interface TSYS.IF configured for:
   - receiving T-REC a training dataset comprising a training medical image IMG-T and related non-imaging data PDAT-T,
   - determining T-DET output data by applying a second machine learning model ML-1 to the training medical image IMG-T, wherein the second machine learning model ML-2 comprises the first machine learning model ML-1,
   - modifying T-MDF a parameter of the second machine learning model ML-2 based on a difference between the non-imaging data PDAT-T and the output data,
   - extracting T-EXT the first machine learning model ML-1 from the second machine learning model ML-2,
   - providing T-PROV the first machine learning model ML-1.
Clause 4.3: The model providing system SYS of clause 4.1 or clause 4.2, comprising additional means for executing the method according to one of clauses 2.1 to 2.3, or being configured for executing the method of one of clauses 2.1 to 2.3.
Clause 4.4: A computer program product comprising instructions which, when the program is executed by a computer, cause the computer to carry out the method of one of clauses 2.1 to 2.3.
Clause 4.5. A computer-readable storage medium comprising instructions which, when executed by a computer, cause the computer to carry out the method of one of clauses 2.1 to 2.3.

Wherever not already described explicitly, individual embodiments, or their individual aspects and features, can be combined or exchanged with one another without limiting or widening the scope of the described invention, whenever such a combination or exchange is meaningful and in the sense of this invention. Advantages which are described with respect to one embodiment of the present invention are, wherever applicable, also advantageous of other embodiments of the present invention.

## Claims

1. A computer-implemented method for providing a similar medical image (IMG-S), comprising:
- receiving (REC-IMG-1) a first medical image (IMG-1) related to a first patient (PAT-1);
- determining (DET-FV-1) a first feature vector (FV-1) by applying a first machine learning model (ML-1) to the first medical image (IMG-1),
wherein the first machine learning model (ML-1) was trained based on training data sets, each training dataset comprising a training medical image (IMG-T) and related non-imaging data (PDAT-T) ;
- receiving (REC-FV-2) a plurality of second feature vectors (FV-2), wherein the plurality of second feature vectors (FV-2) is the result of applying the first machine learning model (ML-1) to a plurality of second medical images (IMG-2),
- determining (DET-FV-S), based on the first feature vector (FV-1), a similar feature vector (FV-S) from the plurality of second feature vectors (FV-2);
- selecting (SEL-IMG-S) the similar medical image (IMG-S) from the plurality of second medical images (IMG-2), wherein the similar medical image (IMG-S) is related to the similar feature vector (FV-S); and
- providing (PROV-IMG-S) the similar medical image (IMG-S).

2. The method according to claim 1, wherein the first medical image (IMG-1) is a histopathology slide image, particularly a whole-slide image.

3. The method of claim 1 or 2, wherein the non-imaging data (PDAT-T) comprises at least one of the following categories:
- omics data,
- diagnosis data,
- treatment history, and
- demographic data.

4. The method of claim 3, wherein the non-imaging data (PDAT-T) comprises omics data, wherein the omics data comprises at least on of
- genomic data,
- transcriptomic data,
- proteomic data, and
- metabolomic data.

5. The method of one of the preceding claims, wherein the first machine learning model (ML-1) was trained by training a second machine learning model (ML-2) comprising the first machine learning model (ML-1), wherein the second machine learning model (ML-2) was trained to predict non-imaging data based on medical images, wherein advantageously the second machine learning model (ML-2) is a classifier.

6. The method of one of the preceding claims, wherein the first machine learning model (ML-1) comprises a feature unit (ML-F) and an attention unit (ML-A), wherein the attention unit (ML-A) is based on an attention mechanism,
wherein the step of determining (DET-FV-1) the first feature vector (FV-1) comprises:
-- determining (DET-SF) a plurality of sub-features by applying the feature unit (ML-F) to the first medical image (IMG-1), and
-- determining (DET-AM) the first feature vector (FV-1) by applying the attention unit (ML-A) to the plurality of sub-features.

7. The method according to claim 6, wherein the step of determining (DET-FV-1) the first feature vector (FV-1) comprises:- reducing (RED-DIM) dimensionality of the sub-features by principal component analysis and/or applying learnable bottleneck layers.

8. The method according to claim 6 or claim 7, wherein the first machine learning model (ML-1) is a Deep Local Feature Network.

9. The method according to one of the preceding claims, furthermore comprising:
- determining (DET-SEG) a segmentation of a tumor within the first medical image (IMG-1),
wherein, in the step of determining (DET-FV-1) a first feature vector (FV-1), the machine learning model is applied to a part of the first medical image (IMG-1) corresponding to the segmented tumor.

10. The method according to one of the claims 1 to 8, comprising:
- dividing (DIV-IMG-1) the first medical image (IMG-1) into a plurality of first patches (PTCH-1),
wherein, in the step of determining (DET-FV-1) the first feature vector (FV-1), the first machine learning model (ML-1) is applied to one of the first patches (PTCH-1).

11. The method according to claim 10, wherein the similar medical image (IMG-S) comprises a plurality of second patches (PTCH-S), wherein one of the second patches (PTCH-S) is related to the similar feature vector (FV-S), and wherein providing (PROV-IMG-S) the similar medical image (IMG-S) comprises displaying an association between the one of the first patches (PTCH-1) and the one of the second patches (PTCH-S).

12. A computer-implemented method for providing a first machine learning model (ML-1), comprising:
- receiving (T-REC) a training dataset comprising a training medical image (IMG-T) and related non-imaging data (PDAT-T),
- determining (T-DET) output data (OUT-T) by applying a second machine learning model (ML-2) to the training medical image (IMG-T), wherein the second machine learning model (ML-2) comprises the first machine learning model (ML-1),
- modifying (T-MDF) a parameter of the second machine learning model (ML-2) based on a difference between the non-imaging data (PDAT-T) and the output data (OUT-T),
- extracting (T-EXT) the first machine learning model (ML-1) from the second machine learning model (ML-2),
- providing (T-PROV) the first machine learning model (ML-1).

13. An image providing system (PSYS) for providing a similar medical image (IMG-S), comprising
- means for receiving (REC) a first medical image (IMG-1) related to a first patient (PAT-1);
- means for determining (DET-FV-1) a first feature vector (FV-1) by applying a first machine learning model (ML-1) to the first medical image (IMG-1),
wherein the first machine learning model (ML-1) was trained based on training datasets, each training dataset comprising a training medical image (IMG-T) and related non-imaging data (PDAT-T) ;
- means for receiving (REC-FV-2) a plurality of second feature vectors (FV-2),
wherein the plurality of second feature vectors (FV-2) is the result of applying the first machine learning model (ML-1) to a plurality of second medical images (IMG-2);
- means for determining (DET-FV-S), based on the first feature vector (FV-1), a similar feature vector (FV-S) from the plurality of second feature vectors (FV-2);
- means for selecting (SEL-IMG-S) the similar medical image IMG-S from the plurality of second medical images (IMG-2), wherein the similar medical image (IMG-S) is related to the similar feature vector (FV-S); and
- means for providing (PROV-IMG-S) the similar medical image (IMG-S).

14. A computer program product comprising instructions which, when the program is executed by a computer, cause the computer to carry out the method of one of the claims 1 to 11.

15. A computer-readable storage medium comprising instructions which, when executed by a computer, cause the computer to carry out the method of one of the claims 1 to 11.
